# EUROPEAN PATENT SPECIFICATION

(11) **EP 3 684 747 B1**
(45) Date of publication and mention of the grant of the patent: **30.08.2023**
(21) Application number: 17926210.0
(22) Date of filing: 21.09.2017
(51) Int. Cl.: C07C 67/08, C07C 67/54, C07C 67/56, C07C 67/60, C07C 69/003

(54) **METHOD OF PREPARING GLYCOL ESTERS WITH LOW COLOR AND PEROXIDE CONTENT**
VERFAHREN ZUR HERSTELLUNG VON GLYKOLESTERN MIT NIEDRIGEM FARB- UND PEROXIDGEHALT
PROCÉDÉ DE PRÉPARATION D'ESTERS DE GLYCOL À FAIBLE TENEUR EN COULEUR ET EN PEROXYDE

(43) Date of publication of application: 29.07.2020
(73) Proprietor: Public Joint Stock Company "Sibur Holding", Tobolsk, Tyumen region 626150 (RU)
(72) Inventor: NOSIKOV, Aleksei Aleksandrovich, s. Turuntaevo 634534 (RU); POPOVTSEV, Egor Evgenievich, Seversk 636000 (RU); IGASHEVA, Varvara Petrovna, s. Porotnikovo 636213 (RU)
(74) Representative: Peters, Andreas
(86) International application number: PCT/RU2017/000691
(87) International publication number: WO 2019/059800

(56) References cited:
- US-A1- 2011 086 997
- US-A1- 2011 086 997
- US-A1- 2011 087 044
- RITSUKO OHURA ET AL: "Effect of Bleaching by Peroxide Bleaching Agent, Influence of Inorganic Builders in Bleaching Bath on the Decomposition of Hydrogen Peroxide and its Bleaching Action", JOURNAL OF HOME ECONOMICS OF JAPAN, vol. 37, no. 3, 1 January 1986 (1986-01-01), pages 183-188, XP055678132,
- RITSUKO OHURA et al.: "Effect of Bleaching by Peroxide Bleaching Agent, Influence of Inorganic Builders in Bleaching Bath on the Decomposition of Hydrogen Peroxide and its Bleaching Action", Journal of Home Economics of Japan, vol. 37, no. 3, 1 January 1986 (1986-01-01), pages 183-188, XP055678132,

## Description

### Field of the invention

The present invention relates to the field of preparation of glycol esters, in particular to triethylene glycol esters of 2-ethylhexanoic acid, used as plasticizers in the manufacture of films for multilayered glass.

### Background of the invention

Glycol esters of organic acids are widely used as plasticizers in various industrial processes. For example, triethylene glycol esters of 2-ethylhexanoic acid or n-heptanoic acid are used as plasticizers in the manufacture of films for multilayered glass. At the same time, such plasticizers must meet high requirements; in particular such characteristics as acidity, color, and peroxide number are important [according to National State Standard 8728-88, p.3, Table 3].

Acidity indicates a residual amount of starting compounds and characterizes the purity of a plasticizer. A high content of acids leads to premature yellowing of articles, thus shortening their service life. The color of a plasticizer, in turn, affects the optical properties of a final product (glass). High color of a plasticizer does not allow production of transparent final products, which significantly limits the field of application of the plasticizer. The peroxide number shows the content of peroxide compounds in a plasticizer, which affect light- and thermal stability of glass since peroxide compounds change their polymer matrix structure when exposed to light and heat, which leads to a reduction in the strength of an article and its premature aging.

Thus, a quality plasticizer must be characterized by as low color, acidity, and peroxide number as possible. Plasticizers used in the manufacture of multilayered glass must have an acidity of not higher than 0.1 mg KOH/g, a peroxide number of not more than 1.50 mM O/kg, and a color value measured on the platinum-cobalt (Pt-Co) scale of not more than 30 units.

One cause of undesired color of glycol esters is associated with side reactions of glycols. The color of glycol esters is reduced by using an ester clarification step.

Methods for reducing the color of esters by using ozone or ozone-containing gases are known from the state of the art. For example, the authors of patent US8524938 disclose a method of clarifying an ester by treating thereof with ozone or ozone-containing gases in an amount of from 0.01 to 5 g of absorbed ozone per liter of ester. The treatment with ozone or ozone-containing gas is carried out at a temperature of 70°C for 0.5 hour at a flow rate of 0.025 m³/hr. After the treatment, the ester is dried at a temperature of 140°C and under a pressure of 1 kPa for 0.5 hour. This provides an ester with a color value of 16 Pt-Co units, an acidity of 0.06 mg KOH/g, and a peroxide number of 1.35 mM O/kg.

Despite achieving a large reduction in the ester color (16 units of the treated ester over 89 Pt-Co units of untreated ester), the ester comprised a large content of peroxide compounds that were formed both at the esterification step and at the clarification step and that deteriorated service properties of the final product. In addition, a disadvantage of this method is a large consumption of an expensive reagent - ozone that is, in addition, toxic for an organism and increases explosion hazard of the industrial process.

Methods of clarifying an ester with a peroxide compound also are known in the state of the art.

Patent CN103012152 discloses, for example, the use of hydrogen peroxide as a clarifying agent. The clarification step is carried out by passing a 30 to 50% solution of hydrogen peroxide through a crude ester, followed by washing the ester with sodium carbonate and water. Then, the ester is clarified by addition of activated carbon.

A mixture of peroxides may be used as a clarifying agent. In patent CN101376631, a diethylene glycol ester of benzoic acid is treated, for example, with a mixture of hydrogen peroxide and sodium percarbonate at a ratio of 7:3 for 0.5 hour. After that, the ester is treated with activated carbon. The ester prepared according to this invention is characterized by a purity of up to 99.5%.

A disadvantage of the technical solutions isclosed in patents CN103012152 and CN101376631 are the absence of the step of decomposition of peroxide compounds, which results in a reduction in the strength characteristics of articles and their premature aging. In addition, said patents do not provide final characteristics of the esters (color, acidity and peroxide number) allowing determination of the field of their application as plasticizers and their effect on the final product.

The document US2011086997 discloses a method for producing glycol esters of organic acids with lower coloration. In accordance with this document, the method includes a step of esterification of glycol and organic acid in the presence of a metal-containing catalyst, followed by distillation of unreacted starting materials. Further stages of the process include treatment of the crude ether with hydrogen peroxide when heated during the clarification stage, neutralization of acidic components, filtration and drying. The disadvantages of this solution are the high content of peroxides in the ester and the need to use additional expensive catalysts.

The combined use of hydrogen peroxide and sodium metasilicate for clarification processes in an alkaline medium is also known (Ritsuko Ohura Effect of bleaching by Peroxide Agent, Influence of Organic Builders in Bleaching Bath on the Decomposition of Hydrogen Peroxide and its Bleaching Action/ / Journal of home economics of Japan, 37, N°3 (1986.01.01), pp 183-188). The use of sodium metasilicate in an alkaline medium leads to an increase in the rate of clarification due to the acceleration of the decomposition of hydrogen peroxide, but the use of sodium metasilicate in the process of hydrogen peroxide does not lead to the decomposition of peroxide in an acidic medium.

To reduce the content of peroxide compounds and, consequently, the peroxide number, one more step is required, wherein decomposition of organic peroxides occurs.

It is known in the state of the art that the following compounds are used for decomposition of organic peroxides: manganese compounds (RU2048454), iron compounds (CN105776624, WO2015194739) and other compounds of transition metals. However, the use of such decomposing agents in the process of clarifying an ester results in an even higher color due to formation of new compounds of transition metals and, therefore, in a necessity of one more step of removing colored impurities.

Thus, at present, a method of preparing glycol esters, which makes it possible to obtain esters with low values of color, acidity and peroxide content is unknown in the background..

### Summary of the invention

The object of the invention is to develop a method of preparing glycol esters suitable for use as plasticizers in the glass industry.

The technical result of the present invention is to prepare an ester with low values of color, acidity, and peroxide content.

The object is addressed and the technical result is achieved by using alkali metal metasilicate together with hydrogen peroxide at the ester clarification step.

The inventors have found that the clarification of ester by using hydrogen peroxide together with alkali metal metasilicate provides an ester with low values of color and peroxide content. Hydrogen peroxide is supposed to convert colored impurities into organic peroxides. The treatment with alkali metal metasilicate, in turn, leads to decomposition of the formed organic peroxides to colorless compounds (acids and alcohols), which, unlike organic peroxides, are easily removed in steps of neutralization and drying.

The method of preparing a glycol ester of an organic acid includes steps of esterification, clarification of the ester, neutralization, filtration and drying.

### Description of drawings

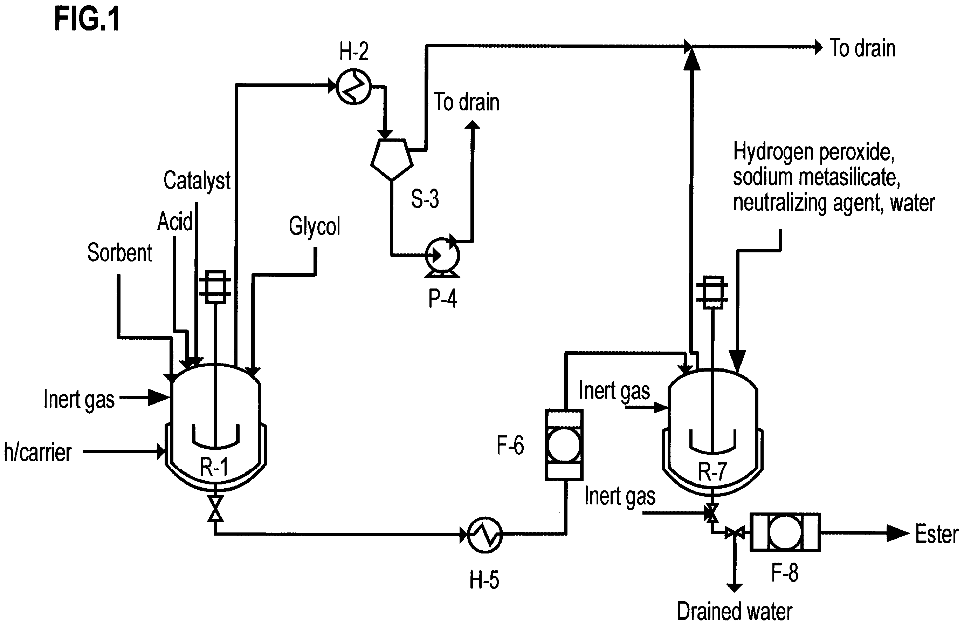

Fig.1 shows a schematic diagram of an apparatus for preparing a glycol ester of an organic acid.

### Detailed description of the invention

The detailed description of various aspects and embodiments of the present invention are given below.

According to the invention, the method of preparing glycol esters of organic acids includes the following steps:
a) esterification of glycol and an organic acid in the presence of a metal-containing catalyst and, optionally, a sorbent to form a crude ester;
a*) filtration from the sorbent if used;
b) clarification of the crude ester by treating thereof with hydrogen peroxide and alkali metal metasilicate at a temperature of from 70 to 200°C and allowing to stand for 0.5 to 1.5 hours to obtain a clarified ester;
c) neutralization of acid compounds (unreacted acid residues and acids formed at step b)) in the clarified ester by treating with an alkaline solution;
d) filtration and drying of the ester to obtain a glycol ester.

### Step a) of esterification

At the esterification step, an ester is formed by reacting glycol with an organic acid in the presence of a metal-containing catalyst and, optionally, a sorbent.

The glycol is a diatomic alcohol comprising up to 20 carbon atoms. Examples of the glycol include, but are not limited to, ethylene glycol, diethylene glycol, triethylene glycol, mono-, di- or tripropylene glycol, butylene glycols, 2,2,4-methylpentanediol, 1,2-cyclohexane dimethanol, and 1,4-cyclohexane dimethanol. Diethylene glycol, dipropylene glycol, and triethylene glycol are preferred.

The organic acid is a linear or branched, aliphatic or aromatic acid comprising from 3 to 20 carbon atoms. Examples of the organic acid include, but are not limited to, heptanoic acid, 2-ethylhexanoic acid, propionic acid, n-butyric acid, isobutyric acid, n-valeric acid, 2-methylbutyric acid, benzoic acid, 2-methylphenylic acid, etc. 2-Ethylhexanoic acid is preferred.

The reversible esterification reaction is shifted toward the reaction products by using one of the starting compounds (as a rule, an organic acid) in excess. The ratio of organic acid:glycol is from 1:1 to 4:1, inclusive, preferably 2.6:1.

The metal-containing catalyst used in the esterification process may be a metal-containing compound known in the state of the art as an esterification catalyst. Examples of such catalysts include, but are not limited to, compounds of titanium, tin, zirconium, and the like. Titanium (IV) compounds, such as titanium tetraisopropoxide, titanium tetraisobutoxide, are preferred, and compositions prepared by the reaction of titanium orthoester with an alcohol, acid and/or base, as disclosed, for example, in EP1035916 and US8232222. The amount of the used catalyst is of from 0.01 to 1 wt.%, inclusive, preferably from 0. 1 to 0.25 wt.%.

At the esterification step, the process of preparing an ester comprises an optional use of sorbents to reduce a color value of the final product due to adsorption of coloring glycol impurities. The sorbents may be any sorbents known in the state of the art, for example activated carbons, zeolites, and perlites. The amount of the used sorbent is of from 0.01 to 2 wt.%, inclusive, preferably from 0.1 to 1 wt.%.

The esterification temperature varies of from 150 to 300°C, inclusive, depending on the activity of the used catalyst; preferably of from 180 to 250°C.

The pressure at the esterification step is from 30 to 100 kPa, inclusive, preferably from 40 to 70 kPa. Such decrease in the pressure at the esterification step is required to increase (intensify) the removal of water that is formed in the reaction and that is a catalyst poison.

The esterification step is performed in an inert gas atmosphere. The inert gas may be nitrogen, argon, helium, carbon dioxide, etc.

The duration of the esterification step is from 3 to 10 hours, inclusive, preferably from 6 to 7 hours.

According to the present invention, the esterification step is performed in a reaction vessel, which is a reactor equipped with a heating element, a mixing device in the form of a mixer or a circulation pump, and a vapor delivery tube used for the removal of the reaction water from the reactor in the form of an azeotropic mixture with an alcohol. At the end of the esterification reaction, the unreacted organic acid is distilled for 0.5 to 2 hours, inclusive, preferably from 0.5 to 1 hour, by reducing the pressure from 40 to 2 kPa, preferably from 7 to 5 kPa.

The mass obtained at the above-described esterification step is a crude ester and comprises the target product - ester, sorbent, catalyst, unreacted acid residues, coloring impurities, and peroxide compounds formed during the esterification.

### Step b) of clarification of the ester

Prior to the clarification step, the obtained mass from step a) of esterification is filtered to separate the sorbent if used.

The clarification step is performed after the esterification step by treating the crude ester with hydrogen peroxide and alkali metal metasilicate.

The alkali metal metasilicate is metasilicate of sodium or potassium, or a mixture thereof. Sodium metasilicate is preferred.

The alkali metal metasilicate is used in the form of a solid salt, including crystalline hydrate of the general formula M₂SiO₃×nH₂O, wherein M is potassium or sodium, n is a number of from 0 to 10, preferably from 7 to 10, more preferably from 8 to 9; and in the form of an aqueous solution at a concentration of from 30 to 60 wt.%, and preferably from 40 to 50 wt.%.

The alkali metal metasilicate is used in an amount of from 0.01 to 2.0 wt.%, inclusive, preferably from 0.05 to 1 wt.%, more preferably from 0.2 to 0.3 wt.%.

Hydrogen peroxide is a colorless liquid, very soluble in water. Hydrogen peroxide may be used in the form of a solution in any commercial form (10%, 30%, 50%). Hydrogen peroxide is used in an amount of from 0.01 to 5 wt.%, inclusive, preferably from 0.05 to 0.1 wt.%.

The addition of alkali metal metasilicate and hydrogen peroxide is carried out in any order, based on the technological convenience, including in a mixture, until the clarification step temperature is reached.

The clarification step temperature is from 70 to 200°C, inclusive, preferably from 100 to 150°C. If the clarification step temperature is less than 70°C, the clarification will be less effective because the oxygen activity in the complex of alkali metal metasilicate and hydrogen peroxide decreases. If the clarification step temperature is higher than 200°C, this causes side oxidation reactions in the system, which, in turn, will lead to an increase in the color of the target ester. In addition, as the temperature increases higher than 200°C, the decomposition rate of the complex of alkali metal metasilicate and hydrogen peroxide increases, thereby reducing the degree of product clarification.

The clarification step is carried out under a pressure of from 50 to 150 kPa, inclusive, preferably from 90 to 110 kPa.

The duration of the clarification step is from 0.5 to 1.5 hours, inclusive, preferably from 1 to 1.16 hours since reaching the above-mentioned temperature. When the duration of the clarification step is less than 0.5 hour, the required reduced color of the target ester is not achieved. When the clarification step lasts more than 1.5 hours, this usually does not give additional advantages in reducing the color of the target product.

The mass obtained after the clarification step contains the target product - ester, catalyst, unreacted acid residues, and a mixture of various organic acids and alcohols formed as a result of successive steps of oxidation with hydrogen peroxide and decomposition of organic peroxides formed at the esterification and oxidation steps with alkali metal metasilicate.

### Step c) of neutralization

The neutralization step is carried out to remove unreacted acid residues and acids formed at the clarification step from the ester.

A neutralizing agent used in the neutralization step is an alkaline solution known in the start of the art, for example, sodium hydroxide, sodium carbonate, etc.

The amount of the neutralizing agent usually ranges from 0.5 to 3 moles per mole of the remaining unreacted acid and acids formed at step b), preferably from 1 to 1.5 moles per mole of the remaining and formed acids. The neutralization process preferably is carried out at a temperature of from 50 to 90°C and under the atmospheric pressure.

At the end of the neutralization step, the mass comprises the target product, hydrolyzed catalyst, salts of organic acids, and water.

### Step d) of filtration and drying

To purify the ester from the residues of the catalyst hydrolyzed at the neutralization step and from salts of organic acids, filtration is carried out in any apparatus known in the state of the art, which are filters equipped with porous filter baffles capable of separating a solid phase from the target product.

Filtration of the crude ester is carried out at a temperature of from 50 to 90°C, inclusive.

The obtained ester is dried to remove therefrom water and/or its vapors. The drying process may be carried out by physical techniques usually used for separation and purification of organic compounds (extraction, desalting, fractional and azeotropic distillation, evaporation, etc.) and by using drying agents that remove moisture by means of adsorption, formation of hydrates or chemical reactions with water.

The drying is preferably performed at a temperature of from 105 to 115°C and under a pressure of from 5 to 10 kPa.

The product obtained in accordance with the present invention is a colorless, transparent oily liquid of a glycol ester of an organic acid.

The invention is shown in more detail in Fig.1, which is a process flow diagram of preparing glycol ester, wherein **R-1** is an esterification reactor equipped with a heating element and a mixing device, **H-2** and **H-5** are condensers, **S-3** is a separator, **P-4** is a pump, **F-6** and **F-8** are filters, and **R-7** is a reactor for clarification of an ester, equipped with a heating element and a mixing device.

The diagram in in the Fig. is an exemplary embodiment of the invention and is not intended to limit thereof.

A glycol ester is prepared by using the following successive steps, wherein:
1) according to the inventive method, an organic acid, glycol, a catalyst, in particular titanium tetraisopropoxide, and optionally a sorbent are supplied to esterification reactor **R-1** equipped with a heating element and a mixing device;
2) further, the reaction mixture of the starting components in reactor **R-1** is heated to the initial temperature of from 180 to 205°C.
3) when the temperature reaches 180 to 205°C, the pressure in the system is lowered from 100 to 70 kPa;
4) when the temperature reach 180 to 205°C and pressure reach 70 kPa, respectively, water and organic acid vapors enter condenser **H-2** through a steam line, where they condense and enter to separator **S-3,** wherein the mixture is separated into two phases: lower aqueous and upper organic phases;
5) the resulting mixture of water and organic acid is periodically removed (drained) from separator **S-3** by using pump **P-4;**
6) to accelerate the process and increase the selectivity of the process of preparing the target product, 0.5 hour after the start of heating, the temperature in esterification reactor **R-1** is increased from 180-205°C to 205-210°C, and 1.5 hours after the start of heating, the temperature is further raised to 215-220°C. The pressure in the system is lowered to 30 kPa;
7) five hours after heating the reaction mass, the esterification reaction is complete. The unreacted acid in the esterification reactor is distilled off. For this, the pressure in separator **S-3** is lowered to 15 kPa by using a vacuum pump and, after another 0.5 hour, the pressure is reduced to 5 kPa. During the pressure reduction, the acid begins to evaporate intensively from reactor **R-1** through a steam line to condenser **H-2** and then to separator **S-3;**
8) six hours after the heating, the reaction mass is discharged from reactor **R-1;**
9) the resulting ester is filtered, if necessary, from sorbents through filter **F-6;**
10) the filtered ester is poured into reactor **R-7** where steps of clarification, neutralization and drying are carried out;
11) hydrogen peroxide and alkali metal metasilicate are added to the ester filtered through filter **F-6;**
12) further, the reaction mass in reactor **R-7** is heated to 100 to 110°C;
13) when temperature reaches 100-110°C, the process of clarification is performed that lasts for one hour;
14) after completion of the clarification step, the neutralization process is performed in reactor **R-7**;
15) during the neutralization process, the ester is treated with an aqueous solution of a neutralizing agent and water to remove the acid residues;
16) further, the ester is dried in **R-7** to remove water residues and is filtered through **F-8** to separate a solid phase (catalyst and salts of organic acids).

The resulting product is identified by IR and NMR spectroscopy methods. The resulting product is a colorless oily liquid - a glycol ester of an organic acid that is characterized by low values of color, acidity, and peroxide number and can be used as a plasticizer in the manufacture of articles, in particular multilayered glass.

In the manufacture of multilayered glass, a plasticizer is used in an amount of from 10 to 60 wt.% as a component of a film fastening silicate glass and organic glass together. A composition and a method of preparing a film for multilayered glass are disclosed in documents WO2009047222, WO2011078313, CN101637991, WO2011006849, etc., wherein glycol ester can be used both as a main plasticizer, and as an additional plasticizer. Films comprising glycol esters may be used not only as an intermediate layer in multilayered glass but also as an adhesive film in a photoelectric module, as disclosed, for example, in RU2600358.

### Embodiments of the invention

### Methods of testing esters

Acidity was determined according to National State Standard 8728-88.

The peroxide content was determined according to National State Standard 26593-85.

The color of an ester was determined according to ASTMD 1209-05.

### IR spectroscopy method (IR-spectroscopy)

A qualitative analysis of the composition of the resulting reaction mass was carried out using IR-spectroscopy by the method of frustrated total internal reflection (FTIR) on an Varian Excalibur HE 3600 spectrometer equipped with the PIKE MIRacle ATR accessory (crystal material - ZnSe/diamond).

The scanning range was 4000 to 400 cm⁻¹, the number of scans was 32, and the spectral resolution was 4 cm⁻¹. Samples were analyzed without processing of the sample.

The resulting product was identified by comparison of the obtained IR spectrum with that of a commercially available sample of triethylene glycol 2-ethylhexanoate.

The band assignment in the IR spectrum: 1731 (-C=O in ester), 1252, 1172, 1120 (-C-O- in ester), 2959, 2873, 1459 (CH₂), 2933, 1382 (CH₃), and 1041 (-C-O in ether).

### NMR spectroscopy method (NMR spectroscopy)

The qualitative composition of the reaction mass samples was determined by the method of high-resolution hydrogen (¹H NMR) nuclear magnetic resonance spectroscopy in a Bruker Avance III spectrometer (400 MHz). For the study, a 30 mg sample was dissolved in 0.6 ml of deuterated chloroform (CHCl₃-d). The number of scans in ¹H nuclei was equal 4.

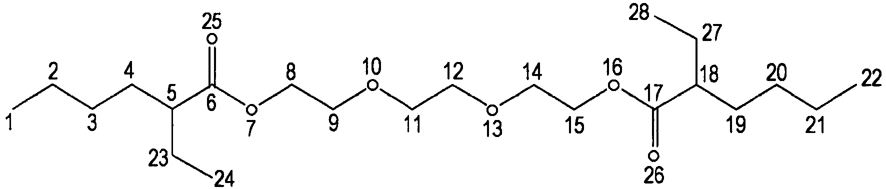

1H NMR spectrum (CDCl₃, δ, m.d., J/Hz): 0.84-0.89 (m, C1H3, C22H3, C24H3, C28H3, J=7.47); 1.19-1.34 (m, C2H2, C3H2, C20H2, C21H2); 1.39-1.66 (m, C4H2, C19H2, C23H2, C27H2, J=7,3); 2.25-2.32 (m, C5H1, C18H1, J=5.36); 3.63 (s, C11H2, C12H2); 3.68 (t, C9H2, C14H2, J=4.87); 4.23 (t, C8H2, C15H2).

### Example 1. Preparation of an ester by using a sorbent, clarification of the ester with sodium metasilicate and hydrogen peroxide

A 15 liter steel reactor equipped with a heating element and a stirring device was charged with 5100 grams of 2-ethylhexanoic acid, 2040 grams of triethylene glycol, 71.4 grams of sorbent, which was activated carbon, and 17.9 grams of titanium (IV) tetraisopropoxide.

The reaction was carried out in the flow of an inert gas, which was nitrogen, at a flow rate of 25 L/h.

The reaction mass was heated to 205°C, and the pressure in the system was lowered to 70 kPa. Then, 0.5 hour after the start of heating, the temperature was raised to 210°C, while reducing the pressure to 60 kPa.

Further, 1.5 hours after the start of heating, the pressure in the reactor was lowered to 40 kPa and the temperature was raised to 215°C.

In order to distill off the unreacted acid, 5 hours after the start of heating, the pressure in the system was lowered to 15 kPa, and after another 0.5 hour, the pressure was reduced to 5 kPa.

Six hours after the start of heating, the reaction mass was cooled.

5500 grams of the reaction mass were discharged from the esterification reactor, the mass containing 3.0 wt.% 2-ethylhexanoic acid, 1.32 wt.% triethylene glycol 2-ethylhexanoate, 3.0% water and 92.0 wt.% triethylene glycol di-2-ethylhexanoate.

Then, the reaction mass pre-filtrated from the sorbent was loaded into a 15liter steel reactor equipped with a heating element. Sodium metasilicate (Na₂SiO₃×9H₂O) and a 37%-solution of hydrogen peroxide (H₂O₂) were successively added to the reaction mass in an amount of 0.2 wt.% and 0.1 wt.%, respectively.

The reaction mass was heated to 105°C with continuous stirring. The heating was lasted for 1 hour.

The purification of the reaction mass, which contained a clarified ester after the clarification step, also included neutralization of the remaining acid and an acid formed at the clarification step with a neutralizing agent, which was an aqueous solution of sodium hydroxide. The amount of sodium hydroxide was from 1 to 1.5 moles per mole of the remaining acid. The neutralization process was carried out at a temperature of 85°C and under the atmospheric pressure for 0.5 hour.

The resulting ester - triethylene glycol di-2-ethylhexanoate was dried at a temperature of from 105 to 115°C and under a pressure of from 10 to 5 kPa to remove therefrom water residues.

The resulting product was filtered off from solid impurities with a filtering agent (Decofill) in an amount of 1 wt.%.

The resulting ester had a color value of 30 Pt-Co units, an acidity of 0.08 mg KOH/g, and a peroxide number of 0.19 mM O/kg.

### Example 2. Preparation of an ester by using a sorbent, clarification of the ester with hydrogen peroxide

The process was carried out as disclosed in Example 1, except that after filtration from the sorbent, a 37% solution of hydrogen peroxide (H₂O₂) was added to the reaction mass in an amount of 0.1 wt.%.

The reaction mass was heated to 120°C with continuous stirring. The heating was lasted for 1 hour. Neutralization and drying were carried out as disclosed in Example 1.

The resulting ester had a color value of Pt-Co 27 units, an acidity of 0.37 mg KOH/g, and a peroxide number of 8.50 mM O/kg.

### Example 3. Preparation of an ester by using a sorbent, clarification of the ester with sodium metasilicate

The process was carried out as disclosed in Example 1, except that after filtration from the sorbent, sodium metasilicate (Na₂SiO₃x9H₂O) was added to the reaction mass in an amount of 0.2 wt.%.

The reaction mass was heated to 105°C with continuous stirring. The heating was lasted for 1 hour. Neutralization and drying were carried out as disclosed in Example 1.

The resulting ester had a color value of Pt-Co 97 units, an acidity of 2.30 mg KOH/g, and a peroxide number of less than 0.10 mM O/kg.

### Example 4 (comparative). Preparation of an ester by using a sorbent, without the clarification step

The reaction mass pre-filtered from the sorbent, obtained after esterification, was loaded into a 15liter steel reactor equipped with a heating element, for subsequent purification from the excess of acid.

The ester-containing reaction mass purification included neutralization of the acid remaining in the ester with a neutralizing agent, which was an aqueous solution of sodium hydroxide. The amount of sodium hydroxide was 1 to 1.5 moles per mole of the remaining acid. The neutralization process was carried out at a temperature of 85°C and under the atmospheric pressure for 0.5 hour.

The resulting ester - triethylene glycol di-2-ethylhexanoate was dried at a temperature of from 105 to 115°C and under a pressure of from 10 to 5 kPa to remove water residues.

The resulting product was filtered off from solid impurities with a filtering agent (Decofill) in an amount of 1 wt.%.

The resulting ester had a color value of 100 Pt-Co units, an acidity of 0.73 mg KOH/g, and a peroxide number of 0.40 mM O/kg.

### Example 5. Preparation of an ester by using a sorbent, the ester clarification step by using hydrogen peroxide, carried out after the acid neutralization step

The reaction mass pre-filtered from the sorbent, obtained after esterification, was loaded into a 15liter steel reactor equipped with a heating element, for subsequent purification from the excess of acid.

The reaction mass purification included neutralization of the acid remaining in the ester with a neutralizing agent, which was an aqueous solution of sodium hydroxide. The amount of sodium hydroxide was 1 to 1.5 moles per mole of the remaining acid.

The neutralization process was carried out at a temperature of 85°C and under the atmospheric pressure for 0.5 hour.

After that, a 37% solution of hydrogen peroxide (H₂O₂) was added in an amount of 0.1 wt.%.

The reaction mass was heated to 105°C with continuous stirring. The heating was lasted for 1 hour.

The resulting ester - triethylene glycol di-2-ethylhexanoate was dried at a temperature of from 105 to 115°C and under a pressure of from 10 to 5 kPa to remove water residues.

The obtained product was filtered off from solid impurities with a filtering agent (Decofill) in an amount of 1 wt.%.

The resulting ester had a color value of 23 Pt-Co units, an acidity of 0.24 mg KOH/g, and a peroxide number of 18.6 mM O/kg.

### Example 6. Preparation of an ester by using a sorbent, the ester clarification step by using hydrogen peroxide and the neutralization step by using sodium hydroxide, carried out simultaneously

The reaction mass pre-filtered from the sorbent, obtained after esterification, was loaded into a 15-liter steel reactor equipped with a heating element, for subsequent purification from the excess of acid.

A 37% solution of hydrogen peroxide (H₂O₂) in an amount of 0.1 wt.% and an aqueous solution of sodium hydroxide were added to the ester. The amount of sodium hydroxide was 1 to 1.5 moles per mole of the remaining acid.

The reaction mass was heated to 105°C with continuous stirring. The heating was lasted for 1 hour.

The resulting ester - triethylene glycol di-2-ethylhexanoate was dried at a temperature of from 105 to 115°C and under a pressure of from 10 to 5 kPa to remove water residues.

The obtained product was filtered off from solid impurities with a filtering agent (Decofill) in an amount of 1 wt.%.

The resulting ester had a color value of Pt-Co 28 units, an acidity of 0.86 mg KOH/g, and a peroxide number of 4.9 mM O/kg.

### Example 7. Preparation of ester by using a sorbent, clarification of the ester with sodium carbonate and hydrogen peroxide neutralization of acid with sodium hydroxide

The process was carried out as disclosed in Example 1, except that after filtration from the sorbent, sodium carbonate (Na₂CO₃)and a 37% solution of hydrogen peroxide (H₂O₂) was added to the ester in an amount of 0.2 wt.% and 0.1 wt.%, respectively.

The reaction mass was heated to 105°C with continuous stirring. The heating was lasted for 1 hour.

Then the resulting ester was treated with a 15% solution of sodium hydroxide in an amount of 1.5 to 2.0 moles per mole of the remaining acid.

The resulting ester - triethylene glycol di-2-ethylhexanoate was dried at a temperature of from 105 to 115°C and under a pressure of from 10 to 5 kPa to remove water residues.

The obtained product was filtered off from solid impurities with a filtering agent (Decofill) in an amount of 1 wt.%.

The resulting ester had a color value of 50 Pt-Co units, an acidity of 1.95 mg KOH/g, and a peroxide number of 1.48 mM O/kg.

### Example 8. Preparation of an ester without using a sorbent, clarification of the ester with sodium metasilicate and hydrogen peroxide

The process was carried out as disclosed in Example 1, except that a sorbent was not added at the esterification step.

Then the ester was loaded into a 15-liter steel reactor equipped with a heating element. Sodium metasilicate (Na₂SiO₃×9H₂O) and a 37%-solution of hydrogen peroxide (H₂O₂) were successively added to the ester in an amount of 0.2 wt.% and 0.1 wt.%, respectively.

The reaction mass was heated to 105°C with continuous stirring. The heating was lasted for 1 hour.

The purification of the reaction mass containing a clarified ester after the clarification step, also included neutralization of the acid remaining after distillation and an acid formed at the clarification step, with a neutralizing agent, which was an aqueous solution of sodium hydroxide. The amount of sodium hydroxide was 1 to 1.5 moles per mole of the remaining acid. The neutralization process was carried out at a temperature of 85°C and under the atmospheric pressure for 0.5 hour.

The resulting ester - triethylene glycol di-2-ethylhexanoate was dried at a temperature of from 105 to 115°C and under a pressure of from 10 to 5 kPa to remove water residues.

The obtained product was filtered off from solid impurities with a filtering agent (Decofill) in an amount of 1 wt.%.

The resulting ester had a color value of 30 Pt-Co units, an acidity of 0.1 mg KOH/g, and a peroxide number of 0.63 mM O/kg.

Table 1 presents the characteristics of the clarified esters obtained in Examples 1-8, as well as the characteristics of the starting, non-clarified ester.

**Table 1. Characteristics of clarified esters**

| Results of the analyses | Example 1 | Example 2 | Example 3 | Example 4 | Example 5 | Example 6 | Example 7 | Example 8 | Starting reaction mass | Norm* |
|---|---|---|---|---|---|---|---|---|---|---|
| Acidity, mg KOH/g of compound | 0.08 | 0.37 | 2.30 | 0.73 | 0.24 | 0.86 | 1.95 | 0.10 | 10.00 | 0.10 |
| Peroxide number, mM O/kg of compound | 0.19 | 8.50 | <0.10 | 0.40 | 18.60 | 4.90 | 1.48 | 0.63 | 5.90 | 1.50 |
| Color, Pt-Co | 30.0 | 27.0 | 97.0 | 100.0 | 23.0 | 28.0 | 50.0 | 30.0 | >505.0 | 30.0 |

| | | | | | | | | | | |
|---|---|---|---|---|---|---|---|---|---|---|
| *Data of the commercially available ester, triethylene glycol di-2-ethyl hexanoate Example 1 Preparation of an ester by using a sorbent, clarification of the ester with sodium metasilicate and hydrogen peroxide Example 2 Preparation of an ester by using a sorbent, clarification of the ester with hydrogen peroxide Example 3 Preparation of an ester by using a sorbent, clarification of the ester with sodium metasilicate Example 4 (comparative) Preparation of an ester by using a sorbent, without the clarification step Example 5 Preparation of an ester by using a sorbent, the ester clarification step by using hydrogen peroxide, carried out after the acid neutralization step Example 6 Preparation of an ester by using a sorbent, the ester clarification step by using hydrogen peroxide and the neutralization step by using sodium hydroxide, carried out simultaneously Example 7 Preparation of an ester by using a sorbent, clarification of the ester with hydrogen peroxide and neutralization of the acid with sodium carbonate Example 8 Preparation of an ester without using a sorbent, clarification of the ester with sodium metasilicate and hydrogen peroxide | | | | | | | | | | |

As can be seen from the data given in Table 1, the use of only sodium metasilicate (Example 3) or only hydrogen peroxide (Example 2) at the clarification step does not provide an ester with required characteristics. To provide an ester with a low content of acid and peroxides and low color, it is necessary to use at the clarification step hydrogen peroxide together with sodium metasilicate, namely, the crude ester must be treated with hydrogen peroxide and alkali metal metasilicate under heating and allowed to stand for a certain period of time (Example 1). The technical result is achieved only with sodium metasilicate used since other compounds similar in nature to sodium metasilicate (strong base and weak acid salts) used at the clarification step as a peroxide decomposing agent do not provide an ester with satisfactory characteristics (Example 7).

## Claims

1. A method of preparing glycol esters of organic acids, including the following steps:
a) esterification of a glycol and an organic acid in the presence of a metal-containing catalyst and, optionally, a sorbent, followed by distillation of an unreacted acid to form a crude ester;
b) clarification of the crude ester to obtain a clarified ester;
c) neutralization of acidic compounds (unreacted acid residues and acids formed at step b)) in the clarified ester by treating with a neutralizing agent;
d) filtration and drying of the ester to obtain a glycol ester,
**characterized in that** the ester is clarified by treating with hydrogen peroxide together with alkali metal metasilicate under heating and allowed to stand.

2. The method according to claim 1, wherein the sorbent is further filtered when used.

3. The method according to claim 1, wherein the alkali metal metasilicate is a compound of the general formula M₂SiO₃ or a mixture thereof, preferably M is sodium or potassium.

4. The method according to claim 1, wherein alkali metal metasilicate is used in an amount of from 5 to 2.0 wt.%, preferably from 0.05 to 1 wt.%, and more preferably from 0.2 to 0.3 wt.%.

5. The method according to claim 3, wherein alkali metal metasilicate is a solid salt, crystalline hydrate or aqueous solution.

6. The method according to any one of claims 3,5 or 6, wherein alkali metal metasilicate is a compound of the general formula Na₂SiO₃×nH₂O, wherein n is a number of from 0 to 10, preferably from 7 to 10, more preferably from 8 to 9.

7. The method according to claim 6, wherein alkali metal metasilicate is an aqueous solution at a concentration of from 30 to 60 wt.%, and preferably from 40 to 50 wt.%.

8. The method according to claim 1, wherein the glycol is a diatomic alcohol comprising up to 20 carbon atoms, preferably the glycol is selected from ethylene glycol, diethylene glycol, triethylene glycol, mono-, di- or tripropylene glycol, butylene glycols, 2,2,4-methylpentanediol, 1,2-cyclohexane dimethanol, and 1,4-cyclohexane dimethanol, more preferably diethylene glycol, dipropylene glycol, and tripropylene glycol.

9. The method according to claim 1, wherein the organic acid is a linear or branched, aliphatic or aromatic acid comprising from 3 to 20 carbon atoms, preferably the organic acid is selected from heptanoic acid, 2-ethylhexanoic acid, propionic acid, n-butyric acid, isobutyric acid, n-valeric acid, 2-methylbutyric acid, benzoic acid, and 2-methylphenylic acid, more preferably the organic acid is 2-ethylhexanoic acid.

10. The method according to claim 1, wherein a ratio of organic acid:glycol is from 1:1 to 4:1, preferably 2.6:1.

11. The method according to claim 1, wherein the metal-containing catalyst is a compound of titanium, tin, or zirconium, preferably a titanium (IV) compound, preferably the titanium compound is titanium tetraisopropoxide, titanium tetraisobutoxide, or a composition prepared by a reaction of titanium orthoester with an alcohol, an acid and/or a base, more preferably the titanium compound is titanium tetraisopropoxide.

12. The method according to claim 11, wherein an amount of the used catalyst is from 0.01 to 1 wt.%, preferably from 0.1 to 0.25 wt.%.

13. The method according to claim 1, wherein the esterification temperature is from 150 to 300°C, inclusive, preferably from 180 to 200°C.

14. The method according to claim 1, wherein the duration of the esterification step is from 3 to 10 hours, inclusive, preferably from 6 to 7 hours.

15. The method according to claim 1, wherein the pressure at the esterification step is from 30 to 100 kPa, inclusive, preferably from 40 to 70 kPa.

16. The method according to claim 1, wherein the sorbent is activated carbon, zeolites, or perlites.

17. The method according to claim 16, wherein an amount of the used sorbent is of from 0.01 to 2 wt.%, preferably from 0.1 to 1 wt.%.

18. The method according to claim 1, wherein the temperature at the clarification step is from 70 to 200°C, inclusive, preferably from 100 to 150°C.

19. The method according to claim 1, wherein the pressure at the clarification step is from 50 to 150 kPa, inclusive, preferably from 90 to 110 kPa.

20. The method according to claim 1, wherein the allowing-to-stand time is from 0.5 to 1.5 hours, inclusive, preferably from 1 to 1.16 hours.

21. The method according to claim 1, wherein hydrogen peroxide is used in an amount of from 0.01 to 5 wt.%, preferably from 0.05 to 0.1 wt.%.

22. The method according to claim 21, wherein hydrogen peroxide is a solution at a concentration of 10%, 30%, or 50%.

23. The method according to claim 1, wherein the neutralizing agent at the neutralization step is an alkaline solution.

24. The method according to claim 1, wherein an amount of the neutralizing agent is from 0.5 to 3 moles per mole of the acid remaining after distillation or the acid formed at step b), preferably from 1 to 1.5 moles per mole of acid.

25. The method according to claim 24, wherein the neutralizing agent at the neutralization step is a solution of sodium hydroxide or sodium carbonate.

26. The method according to claim 1, wherein the neutralization process is carried out at a temperature of from 50 to 90°C, inclusive.

27. The method according to claim 1, wherein the filtration of the crude ester is carried out at a temperature of from 50 to 90°C, inclusive.

28. The method according to claim 1, wherein the drying process is carried out at a temperature of from 105 to 115°C, inclusive.

29. The method according to claim 1, wherein the drying process is carried out under a pressure of from 5 to 10 kPa, inclusive.

## Patentansprüche

1. Verfahren zur Herstellung von Glykolestern von organischen Säuren,
umfassend die folgenden Schritte:
a) Veresterung eines Glykols und einer organischen Säure in der Anwesenheit eines metallhaltigen Katalysators und, gegebenenfalls, eines Sorptionsmittel, gefolgt von der Destillation einer nicht umgesetzten Säure zur Bildung eines rohen Esters;
b) Klärung des rohen Esters zur Gewinnung eines geklärten Esters zu erhalten;
c) Neutralisierung der sauren Verbindungen (nicht umgesetzte Säurereste und in Schritt b) gebildete Säuren) in dem geklärten Ester durch Behandlung mit einem Neutralisationsmittel;
d) Filtration und Trocknung des Esters zur Gewinnung eines Glykolester, **dadurch gekennzeichnet, dass** der Ester geklärt wird durch Behandlung mit Wasserstoffperoxid zusammen mit Alkalimetasilikat unter Erhitzen und Stehenlassen.

2. Verfahren nach Anspruch 1,
wobei das Sorptionsmittel bei der Verwendung weiter gefiltert wird.

3. Verfahren nach Anspruch 1,
wobei das Alkalimetallmetasilikat eine Verbindung der allgemeinen Formel M₂SiO₃ oder ein Gemisch davon ist, vorzugsweise ist M Natrium oder Kalium.

4. Verfahren nach Anspruch 1,
wobei Alkalimetallmetasilikat in einer Menge von 5 bis 2,0 Gew.-%, vorzugsweise von 0,05 bis 1 Gew.-% und besonders bevorzugt von 0,2 bis 0,3 Gew.-% verwendet wird.

5. Verfahren nach Anspruch 3,
wobei das Alkalimetall Metasilikat ein festes Salz, kristallines Hydrat oder eine wässrige Lösung ist.

6. Verfahren nach einem der Ansprüche 3, 5 oder 6,
wobei Alkalimetallmetasilikat eine Verbindung der allgemeinen Formel Na₂SiO₃ xnH₂O, worin n eine Zahl von 0 bis 10, vorzugsweise von 7 bis 10, besonders bevorzugt von 8 bis 9.

7. Verfahren nach Anspruch 6,
wobei das Alkalimetallmetasilikat eine wässrige Lösung mit einer Konzentration von von 30 bis 60 Gew.-% und vorzugsweise von 40 bis 50 Gew.-% ist.

8. Verfahren nach Anspruch 1,
wobei das Glykol ein zweiatomiger Alkohol ist, der bis zu 20 Kohlenstoffatome umfasst, vorzugsweise ist das Glykol ausgewählt ist aus Ethylenglykol, Diethylenglykol, Triethylenglykol, Mono-, Di- oder Tripropylenglykol, Butylenglykol, 2,2,4-Methylpentandiol, 1,2-Cyclohexan-Dimethanol und 1,4-Cyclohexandimethanol, vorzugsweise Diethylenglykol, Dipropylenglykol und Tripropylenglykol.

9. Verfahren nach Anspruch 1,
wobei die organische Säure eine lineare oder verzweigte, aliphatische oder aromatische Säure ist, die 3 bis 20 Kohlenstoffatome umfasst, vorzugsweise ist die organische Säure ausgewählt aus Heptansäure, 2-Ethylhexansäure, Propionsäure, n-Buttersäure Säure, Isobuttersäure, n-Valeriansäure, 2-Methylbuttersäure, Benzoesäure und 2-Methylphenylsäure, vorzugsweise die organische Säure 2-Ethylhexansäure ist.

10. Verfahren nach Anspruch 1,
wobei ein Verhältnis von organischer Säure:Glykol von 1:1 bis 4:1, vorzugsweise 2,6:1, beträgt.

11. Verfahren nach Anspruch 1,
wobei der metallhaltige Katalysator eine Verbindung von Titan, Zinn oder Zirkonium ist, vorzugsweise eine Titan(IV)-Verbindung, vorzugsweise ist die Titan Verbindung Titantetraisopropoxid, Titantetraisobutoxid, oder eine Zusammensetzung, die durch eine Reaktion von Titanorthoester mit einem Alkohol, einer Säure und/oder einer Base hergestellt wird, vorzugsweise ist die Titanverbindung Titantetraisopropoxid.

12. Verfahren nach Anspruch 11,
wobei eine Menge des verwendeten Katalysators von 0,01 bis 1 Gew.-%, vorzugsweise von 0,1 bis 0,25 Gew.-% ist.

13. Verfahren nach Anspruch 1,
wobei die Veresterungstemperatur von 150 bis einschließlich 300°C beträgt, vorzugsweise von 180 bis 200°C.

14. Verfahren nach Anspruch 1,
wobei die Dauer des Veresterungsschritts der Veresterungsstufe 3 bis 10 Stunden beträgt, vorzugsweise von 6 bis 7 Stunden.

15. Verfahren nach Anspruch 1,
wobei der Druck beim Veresterungsschritt 30 bis einschließlich 100 kPa, vorzugsweise 40 bis 70 kPa, beträgt.

16. Verfahren nach Anspruch 1,
wobei das Sorptionsmittel Aktivkohle, Zeolithe oder Perlite ist.

17. Verfahren nach Anspruch 16,
wobei eine Menge des verwendeten Sorptionsmittels von 0,01 bis 2 Gew.-%, vorzugsweise von 0,1 bis 1 Gew.-%, beträgt.

18. Verfahren nach Anspruch 1,
wobei die Temperatur beim Klärungsschritt 70 bis 200°C, vorzugsweise 100 bis 150°C, beträgt.

19. Verfahren nach Anspruch 1,
wobei der Druck in der Klärstufe 50 bis einschließlich 150 kPa, vorzugsweise 90 bis 110 kPa, beträgt.

20. Verfahren nach Anspruch 1,
wobei die Standzeit 0,5 bis einschließlich 1,5 Stunden, vorzugsweise 1 bis 1,16 Stunden, beträgt.

21. Verfahren nach Anspruch 1,
wobei Wasserstoffperoxid in einer Menge von 0,01 bis 5 Gew.-%, vorzugsweise von 0,05 bis 0,1 Gew.-%, verwendet wird.

22. Verfahren nach Anspruch 21,
wobei Wasserstoffperoxid eine Lösung mit einer Konzentration von 10 %, 30 % oder 50 % ist.

23. Verfahren nach Anspruch 1,
wobei das Neutralisationsmittel in der Neutralisationsstufe eine alkalische Lösung ist.

24. Verfahren nach Anspruch 1,
wobei eine Menge des Neutralisationsmittel 0,5 bis 3 Mol pro Mol der nach der Destillation verbleibenden Säure der nach der Destillation verbleibenden Säure oder der in Schritt b) gebildeten Säure beträgt, vorzugsweise von 1 bis 1,5 Mol pro Mol der Säure.

25. Verfahren nach Anspruch 24,
wobei das Neutralisationsmittel in der Neutralisationsstufe eine Lösung von Natriumhydroxid oder Natriumcarbonat ist.

26. Verfahren nach Anspruch 1,
**dadurch gekennzeichnet, dass** der Neutralisationsverfahren bei einer Temperatur von 50 bis einschließlich 90°C durchgeführt wird.

27. Verfahren nach Anspruch 1,
wobei die Filtration des Rohesters bei einer Temperatur von 50 bis einschließlich 90°C durchgeführt wird.

28. Verfahren nach Anspruch 1,
wobei der Trocknungsprozess bei einer Temperatur von 105 bis 115°C, einschließlich, durchgeführt wird.

29. Verfahren nach Anspruch 1,
wobei das Trocknungsverfahren bei einem Druck von 5 bis einschließlich 10 kPa durchgeführt wird.

## Revendications

1. Méthode de préparation d'esters glycoliques d'acides organiques,
comprenant les étapes suivantes
a) estérification d'un glycol et d'un acide organique en en présence d'un catalyseur contenant un métal et, éventuellement, d'un sorbant, suivie de la distillation d'un acide n'ayant pas réagi pour former un ester brut;
b) clarification de l'ester brut pour obtenir un ester clarifié;
c) neutralisation des composes acides non réagis et acides formés à l'étape b)) dans l'ester clarifié par l'ester clarifié en le traitant avec un agent neutralisant ;
d) filtration et séchage de l'ester pour obtenir un ester de glycol, **caractérisé par le fait que** l'ester est clarifié par traitement avec l'eau oxygénée avec du métasilicate de métal alcalin sous l'effet de la chaleur et en le laissant reposer.

2. Méthode selon la revendication 1,
dans lequel l'agent de sorption est encore filtré lors de son utilisation.

3. Méthode selon la revendication 1,
dans lequel le métal alcalin métasilicate est un composé de formule générale M₂SiO₃ ou un mélange de ceux-ci, de préférence M est le sodium ou le potassium.

4. Méthode selon la revendication 1,
dans lequel le métasilicate de métal alcalin est utilisé en une quantité de 5 à 2,0 % en poids, de préférence de 0,05 à 1 % en poids et de manière particulièrement préférée de 0,2 à 0,3 % en poids.

5. Méthode selon la revendication 3,
dans laquelle le métasilicate de métal alcalin est un sel solide, un hydrate cristallin ou une solution aqueuse.

6. Méthode selon l'une des revendications 3, 5 ou 6,
dans laquelle le métasilicate de métal alcalin est un composé de formule Générale Na₂SiO₃ xnH₂O, où n est un nombre de 0 à 10, de préférence de 7 à 10, de préférence de 8 à 9.

7. Méthode selon la revendication 6,
dans laquelle le métasilicate de métal alcalin est une solution aqueuse à une concentration de 30 à 60 % en poids, et de préférence de 40 à 50 % en poids.

8. Méthode selon la revendication 1,
dans laquelle le glycol est un alcool diatomique comprenant jusqu'à 20 atomes de carbone, de préférence le glycol est une alcool diatomique comprenant jusqu'à 20 atomes de carbone, de préférence le glycol est choisi parmi l'éthylène glycol, le diéthylène glycol, le triéthylène glycol, mono-, di- ou tripropylène glycol, butylène glycols, le 2,2,4-méthylpentanediol, le 1,2-cyclohexane diméthanol et le 1,4-cyclohexane diméthanol de préférence diéthylène glycol, dipropylène glycol et tripropylène glycol.

9. Méthode selon la revendication 1,
dans lequel l'acide organique est un acide aliphatique ou aromatique, linéaire ou ramifié, comprenant de 3 à 20 atomes de carbone, de préférence l'acide organique est choisi parmi l'acide heptano que, l'acide 2-éthylhexano que, l'acide propionique, l'acide n-butyrique, l'acide isobutyrique, l'acide n-valérique, l'acide 2-méthylbutyrique, l'acide benzo que et l'acide 2-méthylphénylique, de préférence l'acide organique est l'acide 2-éthylhexano que.

10. Méthode selon la revendication 1,
dans lequel un rapport acide organique:glycol est de 1:1 à 4:1, de préférence de 2,6:1.

11. Méthode selon la revendication 1,
dans lequel le catalyseur contenant du métal est un composé de titane, d'étain ou de zirconium, de préférence un composé de titane (IV), de préférence le composé de titane est le tétraisopropoxyde de titane, le tétraisobutoxyde de titane, ou une composition préparée par une réaction d'orthoester de titane avec un alcool, un acide et/ou une base, de préférence le composé de titane est le tétraisopropoxyde de titane.

12. Méthode selon la revendication 11,
dans lequel une quantité du catalyseur utilisé est de 0,01 à 1 % en poids, de préférence de 0,1 à 0,25 % en poids.

13. Méthode selon la revendication 1,
dans lequel la température d'estérification est de 150 à 300°C inclus, de préférence de 180 à 200°C.

14. Méthode selon la revendication 1,
dans lequel la durée de l'étape d'estérification de l'étape d'estérification est de 3 à 10 heures, de préférence de 6 à 7 heures.

15. Méthode selon la revendication 1,
dans lequel la pression lors de l'étape d'estérification est de 30 à 100 kPa inclus, de préférence de 40 à 70 kPa.

16. Méthode selon la revendication 1,
dans lequel l'agent de sorption est du charbon actif, des zéolithes ou de la perlite.

17. Méthode selon la revendication 16,
dans lequel une quantité de l'agent de sorption utilisé est de 0,01 à 2 % en poids, de préférence de 0,1 à 1 % en poids.

18. Méthode selon la revendication 1,
dans lequel la température lors de l'étape de clarification est de 70 à 200°C, de préférence de 100 à 150°C.

19. Méthode selon la revendication 1,
dans laquelle la pression à l'étape de clarification est comprise entre 50 et 150 kPa inclus, de préférence entre 90 et 110 kPa.

20. Méthode selon la revendication 1,
dans laquelle le temps d'attente est de de 0,5 à 1,5 heure, inclusivement, de préférence de 1 à 1,16 heure. 1 à 1,16 heure.

21. Méthode selon la revendication 1,
dans laquelle le peroxyde d'hydrogène est utilisé à raison de 0,01 à 5 % en poids, de préférence de 0,05 à 0,1 % en poids, à 0,1 % en poids.

22. Méthode selon la revendication 21,
dans laquelle le peroxyde d'hydrogène peroxyde d'hydrogène est une solution à une concentration de 10 %, 30 % ou 50 %.

23. Méthode selon la revendication 1,
dans lequel l'agent de neutralisation dans l'étape de neutralisation est une solution alcaline.

24. Méthode selon la revendication 1,
dans laquelle une quantité de l'agent de neutralisation est de 0,5 à 3 moles par mole de l'acide restant après la distillation de l'acide restant après la distillation ou de l'acide formé à l'étape b), de préférence de 1 à 1,5 mole par mole de l'acide.

25. Méthode selon la revendication 24,
dans lequel l'agent de neutralisation dans l'étape de neutralisation est une solution d'hydroxyde de sodium ou de carbonate de sodium.

26. Méthode selon la revendication 1,
**caractérisé en ce que** le procédé de neutralisation est effectué à une température comprise entre 50 et 90°C inclus.

27. Méthode selon la revendication 1,
dans lequel la filtration de l'ester brut est effectuée à une température comprise entre 50 et 90°C inclus.

28. Méthode selon la revendication 1,
dans lequel le processus de séchage est réalisé à une température comprise entre 105 et 115°C, inclusivement.

29. Méthode selon la revendication 1,
dans lequel le procédé de séchage est effectué à une pression de 5 à 10 kPa inclus.
